# EUROPEAN PATENT APPLICATION

(11) **EP 4 316 474 A1**
(43) Date of publication of application: **07.02.2024**
(21) Application number: 23189109.4
(22) Date of filing: 02.08.2023
(51) Int. Cl.: A61K 31/00, A61K 47/06, A61K 47/14, A61P 17/02

(54) **WOUND TREATMENT TOPICAL COMPOSITION CONTAINING CANNABINOIDS**

(30) Priority: 02.08.2022 US 202263394360 P
(71) Applicant: TF Holdings LLC, Aurora, CO 80010 (US)
(72) Inventor: Fanning, Francis G., Jackson, 83001 (US); Ripsom, Tobias C., Denver, 80218 (US); Peterson, Marnie L., Jackson, 83001 (US); Onyebuagu, William, South Holland, 60473 (US)
(74) Representative: AWA Sweden AB

(57) **Abstract**

A topical composition for dermal wounds or injuries comprising lanolin or derivatives thereof, one or more extracted vegetable oils, one or more extracted plant oils, and one or more cannabinoids. The cannabinoids used in the topical composition may be selected cannabinoids such as CBD and/or analogs and derivatives, THC and/or analogs and derivatives, or mixtures thereof. The extracted vegetable oils and plant oils used in the topical composition may be squalane, meadowfoam seed oil, or a mixture thereof. These components used in the topical composition may be naturally derived from vegetables, plants, or animals. The topical composition has effective pharmacological activity to care for or treat dermal wounds or injuries and may be used to care for or treat a human or an animal.

## Description

### BACKGROUND

There are many different types of dermal wounds and injuries varying in both symptoms and severity. These wounds and injuries may be temporary or permanent, and may be painless or painful. Wounds and injuries may include, for example, sores, infections, lacerations, abrasions, contusions, cuts, surgical incisions, burns, or other topical conditions needing care or treatment.

The pharmaceutical and cosmetic industries have brought many products to market targeting dermal wounds and injuries. Many of these products include compositions and methods having clinical and treatment limitations and cannot care for or treat all types of dermal wounds and injuries.

There is a continuing need for new compositions and methods to better care for or treat dermal wounds and injuries.

### SUMMARY

This disclosure provides embodiments of a topical composition for use on wounds and injuries comprising a combination of a pharmacologically effective amount of lanolin or derivatives thereof, one or more extracted vegetable oils, one or more extracted plant oils, and one or more cannabinoids.

One embodiment is a therapeutic wound treatment topical composition comprising pharmacologically effective amounts of lanolin, meadowfoam seed oil, squalane, and a cannabinoid or mixtures thereof.

In some embodiments, varying amounts of lanolin or derivatives thereof are used. Suitable amounts of lanolin or derivatives thereof include, for example, ranges of about 40-85 wt%, about 50-80 wt%, about 60-80 wt%, or about 70-80 wt%.

In still other embodiments, varying amounts of extracted vegetable oils are used. Suitable amounts of extracted vegetable oils, such as squalane, include ranges of about 1-25 wt%, about 5-20 wt%, about 1-10 wt%, about 2-8 wt%, about 3-7 wt%, or about 1-5 wt%.

In other embodiments, varying amounts of extracted plant oils are used. Suitable amounts of extracted plant oils, such as meadowfoam seed oil, include ranges of about 1-20 wt%, about 4-15 wt%, about 2-10 wt%, about 4-8 wt%, or about 5-7 wt%.

In some embodiments, varying amounts of cannabinoids are used. A person skilled in the art would understand and select amounts of cannabinoids that provide a beneficial biological or clinical activity and a therapeutic window for treating specific or particular dermal wounds and injuries. Suitable amounts of cannabinoids include, for examples, ranges of about 0.05-15 wt%, about 0.25-10 wt%, about 0.25-5 wt%, about 0.5-5 wt%, about 1-5 wt%, or about 1-3 wt%. These cannabinoids may comprise, for example, cannabidiol (CBD including all cannabidiol derivatives), delta-9-tetrahydrocannabinol (THC including all delta-9-tetrahydrocannabinol derivatives), or both CBD and THC. In some embodiments, the CBD comprises about 0.25-10 wt% of the total composition. In other embodiments, the topical composition comprises about 0.25-5 wt% CBD and about 0.25-5 wt% THC.

In some embodiments the topical composition comprises (i) about 0.05-5 wt% by weight of at least one cannabinoid; and (ii) about 1-4 wt% by weight of at least one extracted vegetable oil and one extracted plant oil.

In other embodiments of the topical composition the components comprise one or more of the following features:
(a) the cannabinoid, the extracted vegetable oil, and extracted plant oil comprise a naturally derived product;
(b) the cannabinoid comprises CBD, THC, or CBD and THC;
(c) the CBD comprises about 0.25-5 wt% by weight of the total composition;
(d) the composition comprises about 0.25-5% by weight of CBD and about 0.25-5% by weight of THC;
(e) the extracted vegetable oil and extracted plant oil comprise meadowfoam seed oil and squalane;
(f) the composition comprises about 5-20 wt% of meadowfoam seed oil and about 4-15 wt% of squalane; or
(g) the composition comprises about 0.25-3% by weight of CBD, about 0.25-3% by weight of THC, about 5-20 wt% of meadowfoam seed oil, and about 4-15 wt% of squalane.

Other embodiments of the topical composition described in this disclosure may comprise other components comprising, but not limited to, one or more of a solvent, a thickener, an anti-oxidant, a conditioner, a moisturizer, an emulsifier, a nutrient, an aroma or fragrance, a preservative, a stabilizer, or a combination thereof. These topical compositions may be in the different application forms comprising, for example, an ointment, a cream, an emulsion, a lotion, a paste, an unguent, or a gel, and alternatively, may comprise a waterproof shield on the wound.

A particular embodiment described is a wound treatment composition comprising a pharmacologically effective antimicrobial composition comprising pharmacologically effective amounts of lanolin, meadowfoam seed oil, squalane and cannabinoids; wherein the cannabinoids release cholesterol contained in the lanolin releasing the cholesterol to topically penetrate and treat the wound. A "pharmacologically effective amount" comprises an amount effective to bring about a biological reaction, physiological reaction, a physical change, or a desired clinical benefit or outcome on a wound or injury of a human or animal. Determination of an effective amount is typically within the capacity of persons skilled in the art, especially in light of this disclosure. A "pharmacologically effective amount" will be an amount that provides the desired effect when topically applied to care for or treat dermal wound or injuries.

This disclosure also provides a method of caring for or treating dermal wounds or injuries by applying a therapeutically effective amount of the embodied topical compositions in this disclosure to the skin of a human or an animal in need of care or treatment and the embodied topical composition comprises one or more cannabinoids that release cholesterol contained in the lanolin so that the released cholesterol will topically penetrate skin in order to care for or treat a wound or injury.

### DETAILED DESCRIPTION

This disclosure describes embodiments of a topical composition to care for or treat dermal wounds or injuries comprising lanolin or derivatives thereof, one or more extracted vegetable oils, one or more extracted plant oils, and one or more cannabinoids.

### Lanolin or Derivatives Thereof

Lanolin and derivatives thereof are commercially available materials in a variety of forms for different uses and applications. Briefly, lanolin is a wax ester-a lanolin acid (mixture of many fatty acids) covalently linked to lanolin alcohol (e.g., sterol). Lanolin can act to waterproof hair and skin. Because of its moisturizer or emollient properties (reducing water loss and itching) and antimicrobial actions, lanolin is used in personal care products, such as baby oil, diaper rash products, hemorrhoid medications, lip balm or salve for chapped lips, lotions and skin creams, medicated shampoos, makeup (lipstick, powder, foundation), nipple cream for lactating mothers, and shaving creams among many other products. There are also multiple industrial uses for lanolin including as a lubricant, leather production, textile additive as an emollient making textiles softer, in paints, varnishes, polishes, inks, and as waterproofing for concrete. Lanolin derivatives are also commercially available materials. Derivatives of lanolin, for example, include but are not limited to alcohol cleaved derivatives, fractionated derivatives or saponified derivatives. Persons skilled in the art would understand and selected such lanolin derivatives for use as a wound treatment topical composition according to this disclosure.

In the topical composition of this disclosure, lanolin may provide a source of cholesterol that is released from lanolin alcohol when the described topical composition is applied to cure or treat dermal wounds or injuries. The presence of cholesterol at a wound or injury site provides a biological or clinical benefit during care and treatment of the wound or injury.

### Extracted Vegetable Oils and Plant Oils

This disclosure describes an enhanced efficacy of one or more cannabinoids in a topical composition by formulating them with extracted vegetable oils and plant oils. In one embodiment, pharmacological properties of cannabinoids, such as CBD or THC, alone or together, can be enhanced by combining them with an extracted vegetable oils and plant oils such as, for example, meadowfoam seed oil and squalane. These components may provide beneficial properties and may be used as an emollient, a penetration enhancer, a dispersion agent of the cannabinoids, or a combination of these properties.

Naturally derived squalane, such as hydrogenated squalane, is highly stable to oxidation. It can be found in olives and other vegetable-derived substances, such as, for example, wheat germ. Due to its stability, emollience, and moisturizing effects, squalane can play a valuable role in maintaining the skin barrier film, thereby preventing trans epidermal moisture loss. Squalane is a hydrocarbon that may be derived by hydrogenation of squalene. In contrast to squalene, due to the complete saturation of squalane, it is not subject to auto-oxidation. The hydrogenation of squalene to produce squalane was first reported in 1916. Squalene was traditionally sourced from the livers of sharks. Now, other sources such as olive oil, rice and sugar cane have been used commercially.

Naturally derived meadowfoam seed oil is a high molecular weight plant oil, which can be expressed from the seeds of the Meadowfoam plant (*Limnanthes* Alba). A typical plant profile is: eicosenoic acid C20: 1-61%; docosenoic acid C22: 1-16%; and docosadienoic acid C22: 2-18%. Meadowfoam seed oil typically contains some phytosterols. One of its properties is that it is a liquid at relatively low and ambient temperatures (*i.e.,* room temperatures) and stable at both high and very low temperatures (*i.e*., freezing temperatures). In addition, it readily absorbs into the epidermal region of the skin. As a carrier oil, meadowfoam seed oil is non-comedogenic and does not clog skin pores. It can perform as an emollient and skin softener. It is commonly used in skin care products to help balance sebum production. It can also serve as a 'carrier' for an active ingredient into the epidermal region of the skin.

Squalane and meadowfoam seed oil are high molecular weight, stable, naturally derived plant oils that can enhance the penetration of a topical composition to the outer level of the skin. Exemplified embodiments show that a combination of squalane and meadowfoam seed oil provide, in part, a delivery base for CBD and/or THC. In some embodiments these two naturally derived components will 'surround' or 'encase' the molecules of the CBD and the THC to deliver them 'intact' or 'stable' until they are released onto the skin. Both of the oils can also function to help penetration of the topical composition to the epidermal region of the skin which can deliver enhanced pharmacological efficacy to the site of wounds or injuries in lower levels of the skin.

### Cannabinoids

Cannabinoids are the subject of ongoing research in the pharmaceutical and cosmetic industries. Patents and patent applications in this field include US 10,441,552; US 10,835,504; US 10,864,189; US 11,058,646; US 2016/0120803; US 2020/0352849; US 2021/0046040; and EP 3 875 100. These and other publications disclose the use of cannabinoids in compositions to treat various disorders, such as, toe nail fungus, MRSA infection, herpes virus infection, tinea pedis, burn and other wound infections, sun burn, diabetic infection, eczema, impetigo, dermatophytosis, psoriasis, itchy skin, painful skin, inflamed skin, atopic dermatitis, dandruff, pruritus ani, and general topical infections.

In this disclosure suitable cannabinoids include, but are not limited to, natural phytocannabinoids, organic cannabinoids, endocannabinoids, cannabinoid analogs, cannabinoid derivatives, synthetic cannabinoids and cannabinoid receptor agonists. Those skilled in the art know cannabinoids comprise compounds that interact with a cannabinoid receptor and other cannabinoid mimetics. Examples may include, but not limited to, certain tetrahydropyran analogs (delta-9-tetrahydrocannabinol, delta-8-tetrahydrocannabinol, 6,6,9-trimethyl-3-pentyl-6H-dibenzo[b,d]pyran-1-ol, 3-(1,1-dimethylheptyl)-6,6a7,8,10,10a-hexahydro-1-1hydroxy-6,6-dimythel-9- H-dibenzo[b,d]pyran-9-ol,(-)--(3 S,4S)-7-hydroxy-delta-6-tetrahydrocannabinol-1,1-dimethylheptyl, (+)--(3 S,4S)-7-hydroxy-A-6-tetrahydrocannabinol, and delta-8-tetrahydrocannabinol-11-oic acid); certain piperidine analogs (e.g., (-)--(6S,6aR,9R,10aR)-5,6,6a,7,8,9,10,10a-octahydro-6-methyl-1-3-[(R)-1-m- ethyl-4-phenylbutoxy]-1,9-phenanthridinediol 1-acetate)); certain aminoalkylindole analogs (e.g., (R)-(+)--[2,3-dihydro-5-methyl-3-(4-morpholinylm-ethyl)-pyrrolo[1,2,3,-de- ]-1,4-benzoxazin-6-yl]-1-naphthelenyl-methanone); certain open pyran-ring analogs (e.g., 2-[3-methyl-6-(1-methylethenyl-2-cyclohexen-1-yl]-5-pentyl-1,3-benzendi-o- 1, and 4-(1,1-dimethylheptyl)-2,3'-dihydroxy-6'-a-(3-hydroxypropyl)-1',-2'- ,3',4',5',6'-hexahydrobiphenyl), their salts, solvates, metabolites, and metabolic precursors.

Cannabidiol can be obtained from plant extract, for example hemp, with a trace amount of delta-9-tetrahydrocannabinol or from cannabis extract using a high-cannabidiol cannabis cultivar. Further, cannabinoids are terpenophenolic compounds found in the Cannabis plant belonging to the Cannabaceae family. Cannabinoids can be isolated, for example, by extraction processes or cold pressing from cannabis plants. Plants in the cannabis genus include Cannabis sativa, Cannabis ruderalis, and Cannabis indica. These plants are the natural sources of cannabinoids, a preferred embodiment of this disclosure. Cannabinoids are also available in synthetic forms.

Examples of cannabinoids, cannabinoid analogs and cannabinoid derivatives include, but are not limited to, cannabigerolic acid (CBGA), cannabigerolic acid monomethylether (CBGAM), cannabigerol (CBG), cannabigerol monomethylether (CBGM), cannabigerovarinic acid (CBGVA), cannabigerovarin (CBGV), cannabichromenic acid (CBCA), cannabichromene (CBC), cannabichromevarinic acid (CBCVA), cannabichromevarin (CBCV), cannabidiolic acid (CBDA), cannabidiol (CBD), cannabidiol monomethylether (CBDM), cannabidiol-C.sub.4 (CBD-C.sub.4), cannabidivarinic acid (CBDVA), cannabidivarin (CBDV), cannabidiorcol (CBD-C.sub. 1), delta-9-tetrahydrocannabinolic acid A (THCA-A), delta-9-tetrahydrocannabinolic acid B (THCA-B), delta-9-tetrahydrocannabinol (THC), delta-9-tetrahydrocannabinolic acid-C.sub.4 (THCA-C.sub.4), delta-9-tetrahydrocannabinol-C. sub.4 (THC-C.sub.4), delta-9-tetrahydrocannabivarinic acid (THCVA), delta-9-tetrahydrocannabivarin (THCV), delta-9-tetrahydrocannabiorcolic acid (THCA-C.sub. 1), delta-9-tetrahydrocannabiorcol (THC-C.sub.1), delta-7-cis-iso-tetrahydrocannabivarin, delta-8-tetrahydrocannabinolic acid (.DELTA.sup.8-THCA), delta-8-tetrahydrocannabinol (.DELTA.sup.8-THC), cannabicyclolic acid (CBLA), cannabicyclol (CBL), cannabicyclovarin (CBLV), cannnabielsoic acid A (CBEA-A), cannabielsoic acid B (CBEA-B), cannabielsoin (CBE), cannabinolic acid (CBNA), cannabinol (CBN), cannabinol methylether (CBNM), cannabinol-C.sub.4 (CBN-C.sub.4), cannabivarin (CBV), cannabinol-C.sub.2 (CBN-C.sub.2), cannabiorcol (CBN-C.sub.1), cannabinodiol (CBND), cannabinodivarin (CBVD), cannabitriol (CBT), 10-ethoxy-9-hydroxy-delta-6a-tetrahydrocannabinol, 8,9-dihydroxy-delta-6a-tetrahydrocannabinol, cannabitriolvarin (CBTV), ethoxy-cannabitriolvarin (CB TVE), dehydrocannabifuran (DCBF), cannabifuran (CBF), cannabichromanon (CBCN), cannabicitran (CBT), 10-oxo-delta-6a-tetrahydrocannabinol (OTHC), delta-9-cis-tetrahydrocannabinol (cis-THC), 3,4,5,6-tetrahydro-7-hydroxy-alpha-alpha-2-trimethyl-9-n-propyl-2,6-metha- -no-2H-1-benzoxocin-5-methanol (OH-iso-HHCV), cannabiripsol (CBR) and trihydroxy-delta-9-tetrahydrocannabinol (triOH-THC).

In a particular embodiment of this disclosure, the cannabinoid is a naturally derived product. Naturally derived cannabinoids can be found in a Cannabis Sativa plant variety. Both CBD and THC are commercially available in large quantities. The art has shown that there are cannabinoid receptor sites in the human central nervous system and other tissues. These receptor sites, and their endogenous ligands, comprise the endocannabinoid system. CB1 receptors have also been found in other mammalian tissue, such as in the heart, lung, pituitary glands, etc. CB2 receptor sites can be found in immune cells. The combination of CB1 and CB2 receptors can play an important role in regulating the release of 'messages' to the body, *i.e.,* CB1 receptors from neurons and CB2 receptors from immune cells.

Cannabinoids are usually characterized by low solubility in water and high solubility in lipid mixtures. They do not easily adhere to or remain on skin, and have limited penetration properties. Given these limitations, it is challenging for a skin care formulator to find the best (inactive) co-ingredients, and the best loading requirements, to enhance the efficacy of a cannabinoid (an active ingredient) in the composition when it is applied to and comes in contact with the skin of a human or an animal.

Cannabinoids have anti-inflammatory attributes, which can also help to further moisturize, shrink, plump and smooth skin where needed. (CBD and CBD/THC) are natural plant-based compositions that are useful for treating acne and other skin disorders, while also providing skin rejuvenating properties. When a therapeutically amount of the composition is placed in contact with an area of a skin to be treated, the composition can treat a variety of disorders associated with dermal wounds and injuries.

CBD and/or THC characteristics and properties are unexpectedly enhanced by combining it with meadowfoam seed oil and squalane, which serve as an emollient, penetration enhancer and/or a dispersion agent for the cannabinoid. The anti-microbial and skin metabolic enhancing properties of the embodied compositions make them exceptional dermal care or treatment products that can promote both skin healing and skin rejuvenation.

The topical compositions of this disclosure overcome some of the limitations in skin care products by developing a topical composition comprising lanolin and derivatives thereof, naturally derived cannabinoid(s), naturally derived vegetable oil(s), and naturally derived plant oil(s). One aspect of this disclosure is a formulation having (i) about 0.05-15 wt% of at least one cannabinoid and (ii) about 1-45 wt% of at least one vegetable oil and plant oil. Exemplary cannabinoids include CBD, THC, or CBD and THC. In one embodiment, the CBD comprises about 0.25-10% by weight of the total composition. In another embodiment, the composition comprises about 0.25-5% wt% of CBD and about 0.25-5 wt% of THC.

In particular embodiments, the plant oil comprises meadowfoam seed oil, squalane, or meadowfoam seed oil and squalane. In one formulation, the composition comprises about 5-20 wt% of meadowfoam seed oil and about 4-15 wt% of squalane. In another formulation, the composition comprises about 0.25-3 wt% of CBD, about 0.25-3 wt% by weight of THC, about 5-20 wt% of meadowfoam seed oil, and about 4-15 wt% of squalane.

In other embodiments, the composition further comprises one or more of the following ingredients: a solvent, a thickener, an anti-oxidant, a conditioner, a moisturizer, an emulsifier, a nutrient, an aroma, a preservative, a stabilizer, or a combination thereof.

The anti-microbial compositions and methods of use described herein are helpful for treating a variety of skin disorders, particularly, open wounds or broken, cracked skin conditions.

In embodiments, a topical composition of this disclosure may further comprise an inactive ingredient, such as a solvent, stabilizer, thickener, anti-oxidant, conditioner, moisturizer, preservative, nutrient, fragrance, cationic, ionic and non-ionic surfactant, antipruritic agent, antiperspirant, antipsoriatic agent, antiseborrheic agent, anti-aging agent, anti-wrinkle agent, skin lightening agent, depigmenting agent, or vitamin.

### Additional Embodiments

The topical composition may also further comprise one or more active ingredients, such as an antibiotic, an antiseptic agent, an antifungal, an antibacterial agent, an analgesic, an antiviral agent, an anesthetic, or an anti-cancer agent.

In embodiments, a topical composition can be in the form of a spray, an ointment, a cream, an emulsion, a lotion, a paste, an unguent, or a gel.

Creams according to this disclosure include water-in-oil or oil-in-water emulsions and may further comprise a cleansing agent, an emollient or an aromatic chemical compound.

Ointments and unguents according to this disclosure optionally contain oil and water that can be in a ratio from about 2: 1 to 7:1, and may further comprise a wax, alcohol or petroleum-based mollifying agents.

Gels according to this disclosure optionally contain a vegetable oil up to 5% by weight of the total composition, water and a thickening agent. The thickening agent may be a natural polysaccharide, such as xanthan gum, carrageen, alginate or cellulose gum.

Pastes according to this disclosure may optionally contain aloe gel and beeswax.

Lotions according to this disclosure include oil-in-water or water-in-oil emulsions and may comprise cetyl alcohol, an emulsifier, a fragrance, glycerol, petroleum jelly, a dye, one or more preservatives and/or a stabilizing agent.

Exemplary antibiotics include, but are not limited to, ampicillin, bacampicillin, carbenicillin indanyl, mezlocillin, piperacillin, ticarcillin, amoxicillin-clavulanic acid, ampicillin-sulbactam, benzylpenicillin, cloxacillin, dicloxacillin, methicillin, oxacillin, penicillin G, penicillin V, piperacillin tazobactam, ticarcillin clavulanic acid, nafcillin, procaine penicillin, cefadroxil, cefazolin, cephalexin, cephalothin, cephapirin, cephradine, cefaclor, cefamandole, cefonicid, cefotetan, cefoxitin, cefprozil, cefmetazole, cefuroxime, loracarbef cefdinir, ceftibuten, cefoperazone, cefixime, cefotaxime, cefpodoxime, proxetil, ceftazidime, ceftizoxime, ceftriaxone, cefepime, azithromycin, clarithromycin, clindamycin, dirithromycin, erythromycin, lincomycin, troleandomycin, cinoxacin, ciprofloxacin, enoxacin, gatifloxacin, grepafloxacin, levofloxacin, lomefloxacin, moxifloxacin, nalidixic acid, norfloxacin, ofloxacin, sparfloxacin, trovafloxacin, oxolinic acid, gemifloxacin, perfloxacin, imipenem-cilastatin, meropenem, and aztreonam. In one embodiment, the amount of an antibiotic in a topical composition can be from about 0.01 to 5% by weight of the total composition.

Antiseptic compounds include, but are not limited to, iodine, manuka honey, octenidine dihydrochloride, phenol, polyhexanide, sodium chloride, sodium hypochlorite, calcium hypochlorite, sodium bicarbonate, methyl paraben, benzoyl peroxide and sodium dehydroacetate. In one embodiment, the amount of an antiseptic compound in the topical formulation can be from 0.01 to 5% by weight of the total composition.

Antifungal agents include, but are not limited to, amphotericin B, candicidin, filipin, hamycin, natamycin, nystatin, rimocidin, bifonazole, butoconazole, clotrimazole, econazole, fenticonazole, isoconazole, ketoconazole, luliconazole, miconazole, omoconazole, oxiconazole, sertaconazole, sulconazole, tioconazole, albaconazole, fluconazole, isavuconazole, itraconazole, posaconazole, ravuconazole, terconazole, voriconazole, abafungin, amorolfin, butenafine, naftifine, terbinafine, anidulafungin, caspofungin, micafungin, benzoic acid, ciclopirox, flucytosine, griseofulvin, haloprogin, tolnaftate, undecylenic acid, crystal violet, and balsam of Peru. In one embodiment, the amount of an antifungal agent in the topical formulation can be from 0.01 to 5% by weight of the total composition.

Analgesic agents include, but are not limited to, lidocaine, methyl salicylate, codeine, morphine, methadone, pethidine, buprenorphine, hydromorphine, levorphanol, oxycodone, fentanyl, and non-steroidal anti-inflammatory drugs. The amount of an analgesic agent in the topical formulation can be from about 0.01 to 5% by weight of the total composition.

Anti-viral agents include, but are not limited to, acyclovir, famciclovir, penciclovir, valacyclovir, trifluridine, docosanol, amantadine, rimantadine, oseltamivir, and zanamivir. The amount of an anti-viral agent in the topical formulation can be from about 0.01 to 5% by weight of the total composition.

In some embodiments, the topical composition of this disclosure may further comprise a stabilizer selected from the group consisting of guar gum, xanthan gum cellulose hyaluronic acid, polyvinyl pyrrolidone (PVP), alginate, chondritin sulfate, poly gamma glutamic acid, gelatin, chitosan, corn starch and flour, in an amount from about 0.25 to 2% (w/v)

The art of formulating a skin care composition to treat skin disorders entails the sophisticated application of multiple scientific disciplines, e.g., chemistry, biology, physics, mathematics and materials. One of the common challenges to a formulator is how best to mix an active ingredient(s) with other ingredients (inactive or other active ingredients) to improve the efficacy of the composition. The use of lanolin or a derivative thereof, a naturally derived cannabinoid(s), such as CBD, THC, or a combination of CBD and THC, together with a naturally derived meadowfoam seed oil and squalane, provides a desired beneficial topical formulation.

Formulations may be prepared by conventional procedures known in the pharmaceutical and cosmetic fields. It will be appreciated that the disclosed topical compositions may be varied according to well-known techniques to accommodate differing amounts and types of ingredients. Additionally, the specific components and ingredients and proportions described herein are for illustrative purposes. Ingredients may be exchanged for suitable equivalents or substitutes, and proportions may be varied, according to the desired properties of the dosage form of interest.

The following Examples are intended to illustrate the above disclosure and should not be construed as to narrow its scope. One skilled in the art will readily recognize that the Examples suggest many other ways in which this disclosure could be practiced. It should be understood that numerous variations and modifications may be made while remaining within the scope of this disclosure.

### EXAMPLE

An adolescent female dog went to an animal daycare center and suffered a bloodless but raw puncture wound having flap of skin pulling away from the dome of her head. Rough play with the other dogs at the center likely caused this tear-like wound. A topical composition according to this disclosure comprising CBD was topically applied as a small dollop onto the head of the dog. By the next day all open head wound aspects had sealed shut. Every day gained more and more healing completion with no infection or any other growths/scabs/ or other problematic side effects.

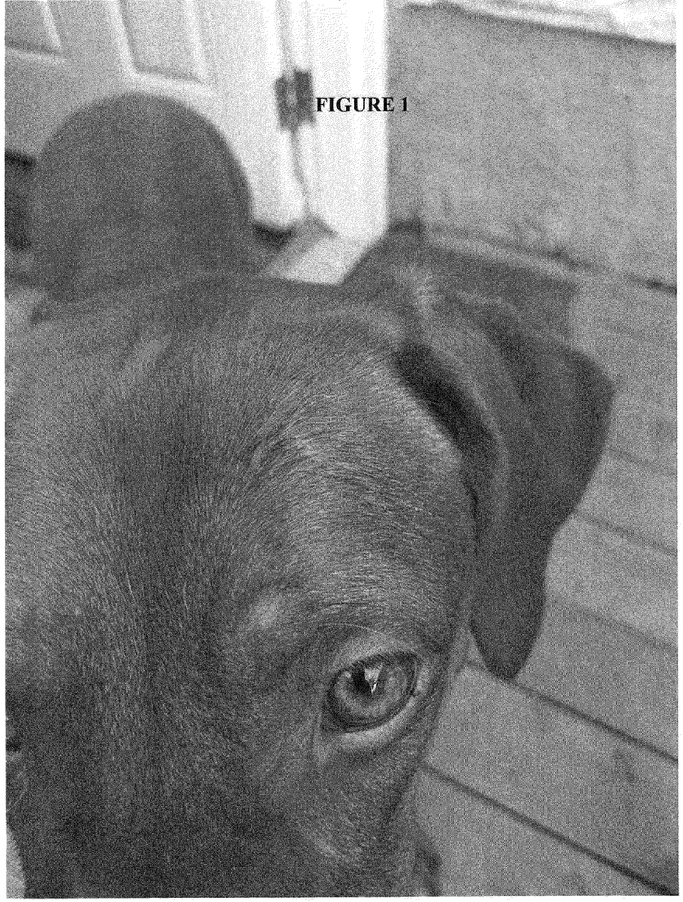

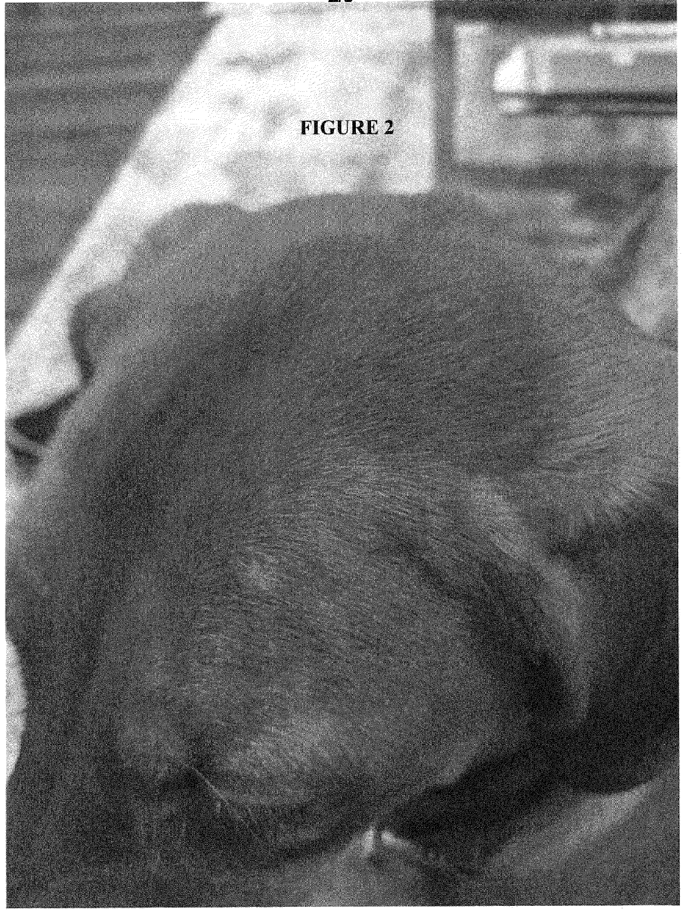

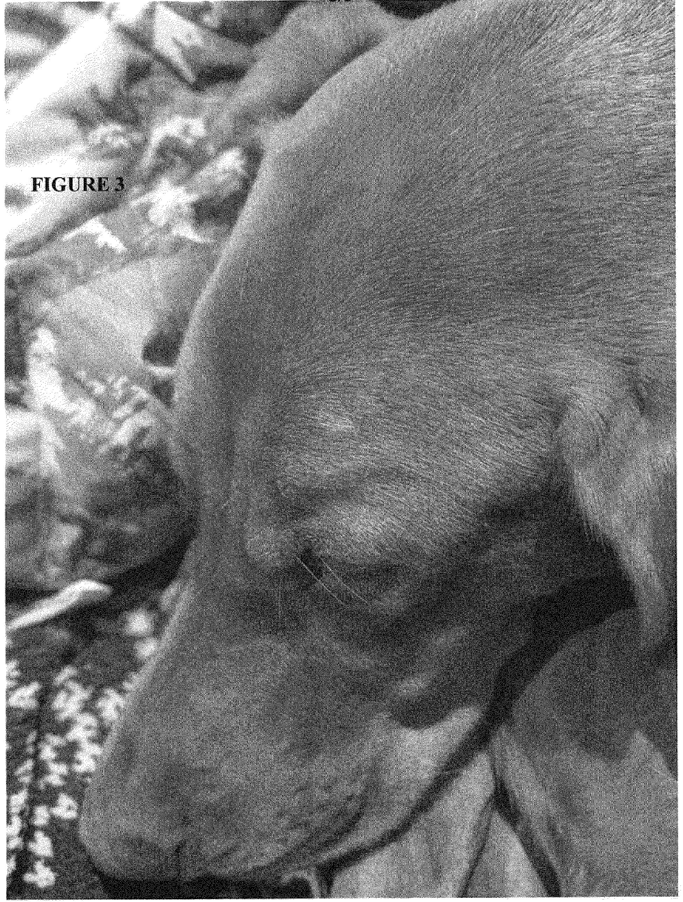

Digital images provided in Figures 1-3 demonstrate the effective wound treatment during the healing process. Figure 1 shows the wound before treatment which is day one of the treatment process. Figure 2 shows the wound on day two. Figure 3 shows the wound after one week. The wound after one week is nicely healed and it looked as if the tear of the fur and skin had never occurred.

Specific embodiments of this disclosure are illustrative and do not limit the scope of this disclosure. Changes and modifications can be made in accordance with ordinary skill in the art without departing from this disclosure in its broader aspects as defined in the following claims.

No limitations inconsistent with this disclosure are to be understood therefrom. This disclosure has been described with reference to various specific and preferred embodiments and techniques. However, it should be understood that many variations and modifications may be made while remaining within the spirit and scope of this disclosure. All publications, patents, and patent documents are incorporated by reference herein, as though individually incorporated by reference.

## Claims

1. A therapeutic wound treatment topical composition comprising a combination of a pharmacologically effective amount of lanolin or derivatives thereof, one or more extracted vegetable oils, one or more extracted plant oils, and one or more cannabinoids.

2. A therapeutic wound treatment topical composition comprising pharmacologically effective amounts of lanolin, meadowfoam seed oil, squalane and cannabinoids.

3. The wound treatment topical composition of claim 1 comprising about 10-85 wt% lanolin or derivatives thereof.

4. The wound treatment topical composition of claim 1 comprising about 40-80 wt% lanolin or derivatives thereof.

5. The wound treatment topical composition of claim 1 comprising about 1-20 wt% meadowfoam seed oil.

6. The wound treatment topical composition of claim 1 comprising about 4-15 wt% meadowfoam seed oil.

7. The wound treatment topical composition of claim 1 comprising about 1-25 wt % squalane.

8. The wound treatment topical composition of claim 1 comprising about 5-20 wt % squalane.

9. The wound treatment topical composition of claim 1 comprising about 0.05-15 wt% cannabinoids.

10. The wound treatment topical composition of claim 1 comprising about 0.25-10 wt% cannabinoids.

11. The wound treatment topical composition of claim 1 comprising about 1-3 wt% cannabinoids.

12. The wound treatment topical composition of claim 1 wherein the cannabinoids comprise THC.

13. The wound treatment topical composition of claim 1 wherein the cannabinoids comprise CBD.

14. The wound treatment topical composition of claim 14 wherein the CBD comprises about 0.25-5 wt% of the total composition.

15. The wound treatment topical composition of claim 1 wherein the topical composition comprises about 0.25-5 wt% CBD and about 0.25-5 wt% THC.

16. The wound treatment topical composition of claim 1 herein the topical composition comprises
(i) about 0.05-15 wt% of at least one cannabinoid; and (ii) about 1-45 wt% of at least one extracted vegetable oil and one extracted plant oil.

17. The wound treatment topical composition of claim 1 wherein the topical composition components comprise one of more of the following features:
(a) the cannabinoid, the extracted vegetable oil, and extracted plant oil comprise a naturally derived product;
(b) the cannabinoid comprises CBD, THC, or CBD and THC;
(c) the CBD comprises about 0.25-10 wt% of the total composition;
(d) the composition comprises about 0.25-5 wt% of CBD and about 0.25-5 wt% of THC;
(e) the extracted vegetable oil and extracted plant oil comprise meadowfoam seed oil and squalane;
(f) the composition comprises about 1-20 wt% of meadowfoam seed oil and about 1-25 wt% squalane; or
(g) the composition comprises about 0.25-5 wt% of CBD, about 0.25-5 wt% of THC, about 4-15 wt% of meadowfoam seed oil, and about 5-20 wt% of squalane.

18. The wound treatment topical composition of claim 1 comprising a spray, an ointment, a cream, an emulsion, a lotion, a paste, an unguent, or a gel.

19. A wound treatment topical composition of claim 1 comprising a pharmacologically effective antimicrobial composition comprising pharmacologically effective amounts of lanolin, meadowfoam seed oil, squalane and cannabinoids; wherein the cannabinoids release cholesterol contained in the lanolin releasing the cholesterol to topically penetrate and treat the wound.

20. A method to topically treat a wound comprising applying the wound treatment topical composition of claim 1 to a subject's wound, wherein the cannabinoids release cholesterol contained in the lanolin releasing the cholesterol to topically penetrate and treat the wound.
